# EUROPEAN PATENT SPECIFICATION

(11) **EP 0 846 269 B1**
(45) Date of publication and mention of the grant of the patent: **24.09.2003**
(21) Application number: 96924657.8
(22) Date of filing: 23.07.1996
(51) Int. Cl.: G01N 33/68, G01N 33/50

(54) **A METHOD OF DETERMINING THE DEGREE OF AGGREGATION OF THE BETA A4 PEPTIDE**
VERFAHREN ZUR BESTIMMUNG DES AGGREGATIONSGRADES DES BETA A4-PEPTIDS
PROCEDE PERMETTANT DE DETERMINER LE DEGRE D'AGREGATION DU PEPTIDE BETA A4

(30) Priority: 16.08.1995 US 515606
(43) Date of publication of application: 10.06.1998
(73) Proprietor: Aventis Pharmaceuticals Inc., Bridgewater, NJ 08807-0800 (US)
(72) Inventor: GOYAL, Shefali, Warren, NJ 07059 (US); PAUL, Joseph, West, Winterville, NC 28590 (US); RIEDEL, Norbert, G., Bedminster, NJ 07921 (US); SAHASRABUDHE, Sudhir, R., Whippany, NJ 07981 (US)
(74) Representative: VOSSIUS & PARTNER
(86) International application number: US9612034
(87) International publication number: WO97007403

(56) References cited:
- EP-A- 0 281 922
- WO-A-95/05604
- WO-A-95/12815
- PROTEIN SCIENCE, vol. 2, 1993, pages 404-410, XP000613188 H. LEVINE: "Thioflavine T interaction with synthetic Alzheimer's disease beta-amyloid peptides: detection of amyloid aggregation in solution." cited in the application
- BRAIN RESEARCH, vol. 420, 1987, pages 233-242, XP000612724 G. PERRY ET AL.: "Antibodies to the neuronal cytoskeleton are elicited by Alzheimer paired helical filament fractions."

## Description

### BACKGROUND OF THE INVENTION

### 1. Field of the Invention

This invention relates of a method of determining the degree of aggregation of the βA4 peptide and more particularly to detecting the protein by reacting the protein with a suitable binding reagent and measuring the amount of resultant unreacted binding reagent.

### 2. Discussion of the Prior Art

It is known that the brain of patients having Alzheimer's Disease contains aggregations or clumps of a small fragment of the beta-amyloid precursor protein which fragment is known as the amyloid beta-peptide or the βA4 peptide. The 42-mer peptide sequence of the βA4 peptide is Aspartate-Alanine-Glutamate-Phenylalanine-Arginine-Histidine-Aspartate-Serine-Glycine-Tyrosine-Glutamate-Valine-Histidine-Histidine-Glutamine-Lysine-Leucine-Valine-Phenylalanine-Phenylalanine-Alanine-Glutamate-Aspartate-Valine-Glycine-Serine-Aspargine-Lysine-Glycine-Alanine-lsoleucine-lsoleucine-Glycine-Leucine-Methionine-Valine-Glycine-Glycine-Valine-Valine-lsoleucine-Alanine. (DAEFRHDSGYEVHHQKLVFFAEDVGSNKGAIIGLMVGGVVIA).

For a general discussion on the βA4 peptide and Alzheimer's Disease, reference is made to Dennis J. Selko, Scientific American , November 1991, 68-78, and Joseph T. Jarrett et al., Cell, Vol. 73, 1055-1058 (1993).

A potential treatment for combating the progress of Alzheimer's Disease is to use a drug comprising an active ingredient which prevents the aggregation or clumping of the βA4 peptide. Accordingly, a screening test for identifying such active ingredient or effective chemical compound is needed.

There are various assays known in the art for detecting proteins per se. One such assay involves the use of Bradford dye, or as it is also known as Coomassie Brilliant Blue G-250. In this regard, reference is made to J. James Sedmack et al., Analytical Biochemistry, 79, 544-552 (1977), which describes an assay for proteins but not for the A4 peptide whether in the aggregate or free state.

G. Johnson, et al. disclose in WO 95/05604, published February 23, 1995, a method for the diagnosis of Alzheimer's Disease. The method utilizes a unique set of proteins which are found to be altered in concentration in patients with Alzheimer's Disease. The invention of Johnson et al. also relates to these proteins and antibodies directed thereto. Kits which can be used for the diagnostic test are also disclosed.

What is required and desired is an assay for the βA4 peptide which distinguishes such protein in the aggregate state from when it is in the free or unaggregated state and reflects the effect of various compounds on such aggregate state. In this regard, H. LeVine, Protein Science, 2, 404-410 (1993) describes the detection of aggregated amyloid employing Thioflavin T.

### BRIEF DESCRIPTION OF THE DRAWINGS

FIG. 1 illustrates the degree of non-aggregation of the βA4 peptide over time.
FIG. 2 illustrates the degree of non-aggregation of the βA4 peptide in terms of a measurable characteristic of the degree of non-aggregation.

### SUMMARY OF THE INVENTION

This invention relates to a method of determining the degree of aggregation of the βA4 peptide and more particularly, the method which comprises reacting the protein with a binding agent which is capable of binding the βA4 peptide only in its non-aggregated state to form an amount of βA4 peptide bound binding reagent. The amount of the reacted binding agent is then measured.

### DETAILED DESCRIPTION

It is known that the brain of Alzheimer's disease patients as compared to the brain of non-Alzheimer's disease individuals, have present an amyloid protein of 39-42 amino acids known as the βA4 peptide. This protein clumps or aggregates in the brain of such Alzheimer's Disease patients whereby the aggregated protein may be responsible for the destruction of normal brain cells. Once the killing clumps or aggregates form, the formation is almost irreversible. Accordingly, a potential treatment of Alzheimer's disease is to treat a patient with a compound or drug which prevents the clump or aggregation of the βA4 peptide.

It has been discovered that potential compounds for treatment of Alzheimer's disease can be identified by a screening test which indicates whether the compounds which are selected as candidates do or do not inhibit the aggregation of the βA4 peptide in vitro.

A binding reagent is selected. The binding reagent is one which selectively reacts with the non-aggregated amyloid peptide, i.e. βA4, but not with the candidate compound being screened, and which in its reacted form, i.e. reacted with the peptide, exhibits a measurable characteristic, e.g. light absorbance at a particular wavelength. The invention discloses the use of binding reagent comprising Coomassie Brilliant Blue G-250. A particularly suitable binding reagent is the Bradford dye. Bradford dye is Coomassie Brilliant Blue G-250. Bradford dye or Coomassie blue dye is described by M. Bradford, Anal. Biochem., 72, 248 (1976); A. H. Reisner et al., Anal. Biochem., 64, 509 (1975); S. Fazekas de St. Groth, et al., Biochim. Biophys. Acta, 71 377 (1963) and J. J. Sedmack et al., Anal. Biochem, 79, 544 (1977), and is commercially available as a standard reagent, (e.g. Protein Assay Dye Reagent Concentrate available from Bio-Rad Life Science Group, Hercules, California). This dye reacts only with non-aggregated βA4 and not with any aggregates of this amyloid peptide.

The Bradford dye has been described, as indicated above, by Marion M. Bradford in Analytical Biochemistry, 72, 248-254 (1976), as 0.01% (weight/volume) (w/v) Coomassie Brilliant Blue G-250, 1.7% (w/v) ethanol, and 8.5% (w/v) phosphoric acid. The Protein Assay Dye Reagent Concentrate, commercially available from Bio-Rad as catalog number 500-0006, is a modified version of the Bradford dye with approximately 0.04% (w/v) Coomassie Brilliant Blue G-250 dissolved in 25% methanol, 50% phosphoric acid and 25% water. The modifications render it more stable, with longer shelf-life, and less bio-hazardous due to decreased phosphoric acid content, without adversely affecting its protein binding properties. It is also available in a kit form with one of two standards, Kit I (with bovine gamma globulin) catalog number 500-0001, or as Kit II (with bovine serum albumin) catalog number 500-0002.

In practice a known concentration of amyloid peptide, i.e. A4 peptide, in its non-aggregated form, is prepared. The βA4 amyloid peptide in its non-aggregated state is obtained by peptide synthesis using a conventional peptide synthesizer, e.g. a MILLIPORE™ Model 9050 peptide synthesizer. For example 10 milligrams of peptide is dissolved in a suitable organic solvent such as dimethylsulfoxide (DMSO) or acetonitrile at a suitable temperature, e.g. 20 to 25°C, to form a stock solution, e.g. 2,500mM. The stock solution, is typically diluted, e.g. ten-fold, in phosphate-buffered saline (pH 7.4) to make a control solution, e.g. of 250mM. A first aliquot, e.g. 16mL, of the control solution is taken, typically with 144mL of the phosphate buffered saline, and reacted with typically 25mL of suitable binding reagent, e.g. Bradford dye, whereby the binding reagent reacts with the non-aggregated amyloid protein to form a first concentration or amount of reacted binding reagent. The reaction with the binding agent is conducted under conditions whereby the binding agent will selectively bind only to the non-aggregated protein. Accordingly, the reaction conditions will be dictated by the particular binding agent employed and what binding characteristic is to be measured. For example, where Bradford dye is employed the binding reaction is typically carried out at a temperature of 20 to 25°C for 5 to 15 minutes in a neutral or slightly basic ambient, that is at a pH ranging from 7.0 to 7.4.

It is to be noted that the first concentration or amount of protein bound binding reagent is measured by a suitable detecting means which is dependent upon the characteristic which the reacted binding agent exhibits, e.g. 0.7 absorbance at a wavelength of 595 nm for Bradford dye, to give a first value X₁.

A second aliquot of the control solution is selected. Since aggregation of βA4 occurs with time, the second aliquot is allowed to incubate at a suitable temperature, e.g. about 37°C, for a suitable time period, e.g. 24 to 72 hours, to form aggregates thereof, whereafter the binding reagent is added thereto and reacts only with the non-aggregated amyloid peptide to form a second concentration or amount of protein bound or reacted binding reagent. The aggregated concentration or amount of amyloid peptide does not react with the binding agent and thus is not detected and measured. Again, the amount of protein bound binding agent is measured, e.g. by absorbance spectroscopy at a suitable wavelength, e.g. 595 nm for Bradford dye, at room temperature to obtain a second value X₂, representing the amount of binding reagent which has reacted with the non-aggregated amount of βA4.

As aggregation occurs, the concentration of protein bound or reacted binding reagent is inverse to the degree of aggregation which has occurred. Accordingly, the second value X₂ is less than X₁, indicating that a certain degree of aggregation of the A4 peptide has occurred.

For purposes of a qualitative screen for candidate Alzheimer's compounds, a third equal aliquot of the control solution is taken. The third aliquot is again allowed to incubate under suitable conditions in the presence of candidate compound, e.g. for 48 hours at 37°C, whereafter the suitable binding agent, e.g. Bradford dye, is added thereto and the resultant solution or mixture is measured, e.g. spectroscopically with a conventional spectrophotometer, to obtain a value of bound binding reagent. If the candidate compound has no anti-aggregating effect then the measured value X₃ , will approximately be equal to the second value, X₂. If on the other hand the third value X₃ exhibits a thirty to forty percent (30-40%) increase over that of X₂ then the candidate compound is considered to be a compound which inhibits aggregation of the amyloid peptide.

In carrying out the above-screening tests, the concentration of amyloid peptide to selected binding reagent should typically range from 1500 to 1800 (amyloid peptide/binding reagent). The concentration of amyloid peptide to candidate compound should typically range from 4 to 40 times or fold (candidate compound/amyloid peptide).

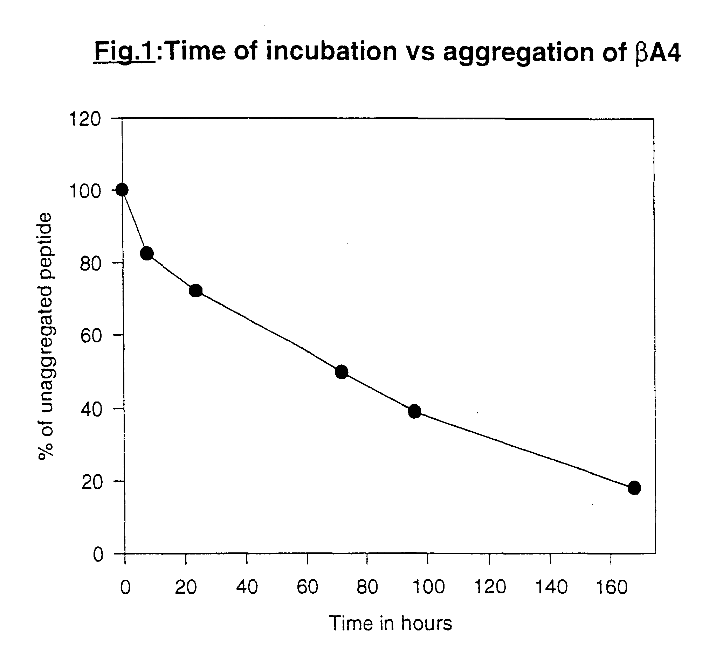

The degree of aggregation can be quantitatively determined in the following manner. Equal aliquots of the control solution are first incubated for various time periods. As the time increases the degree of aggregation increases. The binding agent, e.g. Bradford dye, is added after each time period and the measurement is made, e.g. measuring absorbance at a wavelength of 595 nm for Bradford dye. Thereafter the concentration or percentage of aggregated amyloid protein versus non-aggregated amyloid protein is determined for each time period. The binding agent measures the non-aggregated (or aggregated) protein. The amount of aggregation of β-amyloid is calculated by subtracting this number from that of the fresh protein (unaggregated, X₁ ) and expressed as a percentage. A linear inverse plot of percentage of aggregated peptide is obtained over various time periods, e.g. 24, 48, 72, 96 hours as typically shown in FIG. 1. The procedure is repeated except that after each incubation period the binding agent is added to the incubated aliquot and the resultant mixture is measured to determine the X values. Thereby a correlation between aggregation or non-aggregation can then be established, in terms of an X value, in relation to the spectroscopic reading, e.g. absorbance at a wavelength of 595 nm for Bradford dye as illustrated by FIG. 1. A standard plot is then obtained of X reading to percent of non-aggregation whereby the percent of aggregation is inversely obtained, as typically illustrated by FIG. 1.

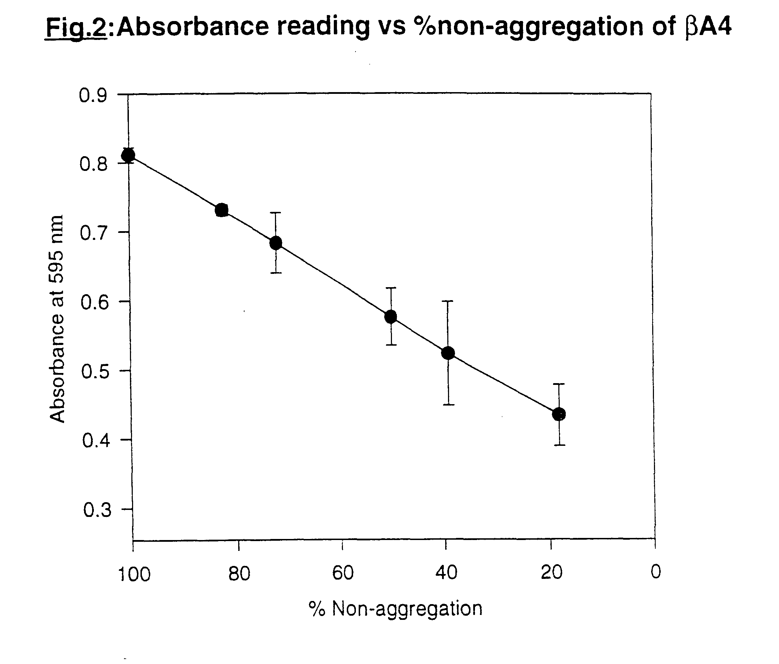

The X values for a particular candidate compound can then be quantitatively determined in terms of percent inhibition of aggregation by comparison with the control, as typically illustrated in FIG. 2.

### Example I

β-amyloid (also known as βA4) peptide was dissolved in 100% dimethylsulfoxide (DMSO) at 10 mg/mL (or 2500 µM) concentration. The resultant solution was diluted to 1 mg/ml (or 250 µM) stock in Phosphate Buffered Saline (PBS), pH 7.4, just prior to setting up the assay and dispensed in individual sample wells of a Corning 96-well plate at 16µL per well (i.e. a final concentration of 25 µM). All treatments were done in triplicate.

In this example, the candidate compound was added to the test wells at three different concentrations (250µM, 500µM and 1mM). Total volume in each well was brought up to 160µL with PBS. For untreated control wells, no compound was added to the β-amyloid peptide, and the total volume was made to 160µL with PBS. The plates were sealed with parafilm and incubated at 37°C for 48 hours.

At the end of the incubation period, the plates were taken out and 25µL of Bio-Rad protein assay dye reagent (Bradford dye) was added to all the wells with samples. A standard curve was also set up at this time for estimation of fresh (also considered as unaggregared) peptide absorbance. The dye was mixed by pipeting and the plates quickly spun at 2500 rpm to remove bubbles.

The absorbance was read at 595 nm in a Dynatech MR5000 plate reader 15 minutes after adding the dye. The percent decrease in aggregation of β-amyloid due to the addition of candidate compound A41920t which is a 8-mer peptide of sequence Glutamine-Lysine-Leucine-Valine-Threonine-Threonine-Alanine-Glutamate (QKLVTTAE) was calculated from the difference between untreated aggregated peptide and the treated aggregated peptide. The percent decrease found was 48.1% with 250 µM of A41920t, 52.44% with 500 µM and 32.26% with 1 mM (or 1000 µM).

### Example 2

The procedure of Example 1 was repeated except that hydrogen peroxide (10µL of 30% stock) was added to the wells in a 96-well plate with 16µL of the 1 mg/mL β-amyloid stock. The final volume was then brought up to 160µL by adding 134µL of PBS to these wells. The plate was sealed and incubated at 37°C for 48 hours.

At the end of the incubation period, the plates were taken out and 25µL of Bio-Rad protein assay dye reagent (Bradford dye) was added to all the wells with samples as in Example 1. A standard curve was again used for estimation of fresh (also considered as unaggregated) peptide absorbance. The dye was mixed by pipeting and the plates quickly spun at 2500 rpm to remove bubbles.

The absorbance was read at 595nm in the Dynatech MR5000 plate reader 15 minutes after adding the dye. The amount of aggregation was estimated by subtracting background and taking the difference between the fresh and hydrogen peroxide treated β-amyloid readings. The difference was expressed as percent over fresh or unaggregated peptide. The percentage of aggregation found was 97.7% after 48 hrs.

### Example 3

The procedure of Example 2 was repeated except that the glucoseamine glycan, pentosan polysulfate was substituted for hydrogen peroxide. The percentage of increase of aggregation found was 40.8% with 0.5 µM, 57.1% with 5 µM and, 62.9% with 50 µM.

### Example 4

The procedure of Example 1 was repeated except that 1-(5'-oxohexyl)-3-methyl-7-propyl-2,6-(1H,3H)-purinedione, also known as propentofylline was substituted for A41920t. The percentage of decrease of aggregation found was 29.68% with 500 µM and 37.4% with 1 mM (or 1000 µM).

### SEQUENCE LISTING

(1) GENERAL INFORMATION:
   (i) APPLICANT:
      (A) NAME: Hoechst Marion Roussel, Inc.
      (B) STREET: 2110 E. Galbraith Rd., P.O. Box 156300
      (C) CITY: Cincinnati
      (D) STATE: Ohio
      (E) COUNTRY: United States of America
      (F) POSTAL CODE (ZIP): 45215-6300
      (G) TELEPHONE: 513-948-7369
      (H) TELEFAX: 513-948-7961
      (I) TELEX: 214320
   (ii) TITLE OF INVENTION: A Method of Determining the Degree of Aggregation of the βA4 Peptide
   (iii) NUMBER OF SEQUENCES: 1
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.30 (EPO)
   (vi) PRIOR APPLICATION DATA:
      (A) APPLICATION NUMBER: US 08/515,606
      (B) FILING DATE: 16-AUG-1995
(2) INFORMATION FOR SEQ ID NO:1:
   (i) SEQUENCE CHARACTERISTICS:
      (A) LENGTH: 42 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:1:

## Claims

1. A method of determining the degree of aggregation of a βA4 peptide (SEQ ID NO: 1) which comprises:
(a) reacting a sample containing free βA4 peptide with a binding reagent comprising Coomassie Brilliant Blue G-250 dye at a temperature of 20 - 25°C for 5 - 15 minutes at a pH ranging from 7.0 to 7.4 to form an amount of non-aggregated βA4 peptide bound to the binding reagent ;
(b) measuring said reacted binding reagent from step(a) to obtain a measurement which correlates to the amount of non-aggregated βA4 peptide present.

2. A method of determining a compound which inhibits the aggregation of a βA4 peptide (SEQ ID NO: 1) which comprises:
(a) reacting a sample containing free βA4 peptide with a binding reagent comprising Coomassie Brilliant Blue G-250 dye at a temperature of 20 - 25°C for 5 - 15 minutes at a pH ranging from 7.0 to 7.4 to form an amount of non-aggregated βA4 peptide bound to the binding reagent;
(b) measuring said reacted binding reagent from step(a) to obtain a first reference measurement which correlates to the amount of non-aggregated βA4 peptide present;
(c) reacting a second sample containing free βA4 peptide with a selected candidate compound for a suitable period of time to form a test sample and adding a binding reagent comprising Coomassie Brilliant Blue G-250 dye at a temperature of 20 - 25°C for 5 - 15 minutes at a pH ranging from 7.0 to 7.4 to form an amount of non-aggregated βA4 peptide to the binding reagent in the test sample; and
(e) measuring said test sample to obtain a second reference measurement to determine the degree of difference between said first and said second reference measurements.

## Patentansprüche

1. Verfahren zur Bestimmung des Aggregationsgrads eines βA4-Peptids (SEQ ID NO: 1), umfassend:
(a) Umsetzen einer Probe, die freies βA4-Peptid enthält, mit einem Bindungsreagens, umfassend einen Coomassie-Brilliant-Blau-G-250-Farbstoff, bei einer Temperatur von 20 bis 25°C für 5 bis 15 Minuten bei einem pH-Wert im Bereich von 7,0 bis 7,4 zur Bildung einer Menge an nicht-aggregiertem βA4-Peptid, das an das Bindungsreagens gebunden ist;
(b) Messen des umgesetzten Bindungsreagenses aus Schritt (a) zum Erhalt einer Messung, die mit der Menge an vorhandenem nicht-aggregiertem βA4-Peptid korreliert.

2. Verfahren zur Bestimmung einer Verbindung, die die Aggregation eines βA4-Peptids (SEQ ID NO: 1) hemmt, umfassend:
(a) Umsetzen der Probe, die freies βA4-Peptid enthält, mit einem Bindungsreagens, umfassend einen Coomassie-Brilliant-Blau-G-250-Farbstoff, bei einer Temperatur von 20 bis 25°C für 5 bis 15 Minuten bei einem pH-Wert im Bereich von 7,0 bis 7,4 zur Bildung einer Menge an nicht aggregiertem βA4-Peptid, das an das Bindungsreagens gebunden ist;
(b) Messen des umgesetzten Bindungsreagenses aus Schritt (a) zum Erhalt einer ersten Referenzmessung, die mit der Menge an vorhandenem nicht-aggregiertem βA4-Peptid korreliert;
(c) Umsetzen einer zweiten Probe, die freies βA4-Peptid enthält, mit einer ausgewählten Kandidatenverbindung über einen geeigneten Zeitraum zur Bildung einer Testprobe und Hinzufügen eines Bindungsreagenses, umfassend einen Coomassie-Brilliant-Blau-G-250-Farbstoff, bei einer Temperatur von 20 bis 25°C für 5 bis 15 Minuten bei einem pH-Wert im Bereich von 7,0 bis 7,4 zur Bildung einer Menge an nicht-aggregiertem βA4-Peptid, zu dem Bindungsreagens in der Testprobe; und
(d) Messen der Testprobe zum Erhalt einer zweiten Referenzmessung zur Bestimmung des Unterschieds zwischen der ersten und der zweiten Referenzmessung.

## Revendications

1. Procédé de détermination du degré d'agrégation d'un peptide βA4 (SEQ ID NO:1) qui comprend :
(a) la réaction d'un échantillon contenant un peptide βA4 libre avec un réactif de liaison comprenant du colorant bleu brillant de Coomassie G-250 à une température de 20-25°C pendant 5-15 min à un pH de 7,0 à 7,4 pour former une quantité de peptide βA4 non agrégé lié au réactif de liaison ;
(b) la mesure dudit réactif de liaison qui a réagi, provenant de l'étape (a), pour obtenir une mesure qui est corrélée à la quantité de peptide βA4 non agrégé présent.

2. Procédé de détermination d'un composé qui inhibe l'agrégation d'un peptide βA4 (SEQ ID NO:1) qui comprend :
(a) la réaction d'un échantillon contenant un peptide βA4 libre avec un réactif de liaison comprenant du colorant bleu brillant de Coomassie G-250 à une température de 20-25°C pendant 5-15 min à un pH de 7,0 à 7,4 pour former une quantité de peptide βA4 non agrégé lié au réactif de liaison ;
(b) la mesure dudit réactif de liaison qui a réagi, provenant de l'étape (a), pour obtenir une première mesure de référence qui est corrélée à la quantité de peptide βA4 non agrégé présent ;
(c) la réaction d'un second échantillon contenant un peptide βA4 libre avec un composé candidat choisi pendant une durée appropriée pour former un échantillon test et l'addition d'un réactif de liaison comprenant du colorant bleu brillant de Coomassie G-250 à une température de 20-25°C pendant 5-15 min à un pH de 7,0 à 7,4 pour former une quantité de peptide βA4 non agrégé au réactif de liaison dans l'échantillon test ; et
(d) la mesure dudit échantillon test pour obtenir une seconde mesure de référence pour déterminer le degré de différence entre lesdites première et seconde mesures de référence.
